# EUROPÄISCHE PATENTANMELDUNG

(11) **EP 4 751 706 A1**
(43) Veröffentlichungstag der Anmeldung: **03.06.2026**
(21) Anmeldenummer: 24216638.7
(22) Anmeldetag: 29.11.2024
(51) Int. Cl.: A61K 8/31, A61K 8/37, A61K 8/46, A61K 8/92, A61Q 1/02, A61K 8/06

(54) **BESCHICHTUNGSMASSE, FOLIE MIT BESCHICHTUNG UND VERFAHREN ZUR HERSTELLUNG DER BESCHICHTUNGSMASSE SOWIE DER FOLIE**

(71) Anmelder: Faber- Castell AG, 90546 Stein (DE)
(72) Erfinder: Beck, Udo, 90461 Nürnberg (DE); Hagge, Anne, 90449 Nürnberg (DE); Häuser, Tobias, 91315 Höchstadt a. d. Aisch (DE); von Godin, Harald, 90522 Oberasbach (DE)
(74) Vertreter: Meissner Bolte Nürnberg

(57) **Zusammenfassung**

Die Erfindung betrifft eine Beschichtungsmasse zum Aufbringen auf eine Folie enthaltend mindestens ein Wachs, wobei das Wachs ausgewählt ist aus einem gemäß EU Verordnung (EG) Nr. 1223/2009 über kosmetische Mittel zugelassenen Wachs, mindestens einen Farbstoff, wobei der Farbstoff ausgewählt ist aus der Liste der in kosmetischen Mitteln zugelassenen Farbstoffe gemäß EU Verordnung (EG) Nr. 1223/2009, mindestens einen Emulgator, wobei der Emulgator ausgewählt ist aus einem gemäß EU Verordnung (EG) Nr. 1223/2009 über kosmetische Mittel zugelassenen Emulgator, mindestens einen pH-Regulator und Wasser. Ferner betrifft die Erfindung eine Folie mit einer Beschichtung aus einer solchen Beschichtungsmasse sowie ein Verfahren zur Herstellung der Beschichtungsmasse sowie der Folie.

## Beschreibung

Die Erfindung betrifft eine Beschichtungsmasse zum Aufbringen auf eine Folie, eine Folie mit einer Beschichtung, die durch Aufbringen der Beschichtungsmasse gebildet ist, sowie ein Verfahren zur Herstellung der Beschichtungsmasse und der Folie.

Zum Anfertigen von Tattoos werden seit langem Folien, sogenannte Stencil-Folien, eingesetzt, die es ermöglichen, ein Design oder eine Zeichnung als Vorlage auf die Haut zu übertragen, nach welcher dann der Tätowierer das eigentliche Tattoo sticht. Die Folien sind hierzu auf einer der Flachseiten beschichtet. Zum Übertragen des Designs auf die Haut gibt es grundsätzlich zwei Möglichkeiten. Zum einen kann das Design oder die Zeichnung direkt auf die der Beschichtung abgewandten Flachseite gezeichnet werden, wobei die Zeichnung durch den Andruck, z.B. mittels eines Bleistiftes, auf ein unter der Folie liegendes Papier, das sogenannte "Transfer-Papier" übertragen wird. Zum anderen kann ein Design in Form einer Druckvorlage, die auf die nicht beschichtete Flachseite der Folie aufgelegt wird, mit Hilfe eines Thermodruckers auf ein Transfer-Papier übertragen werden, welches auf der beschichteten Flachseite der Folie angeordnet wird. Im Thermodrucker erwärmt sich die Stencil-Folie an den Stellen, an denen sich schwarze Bereiche des Designs befinden, sodass die Beschichtung an diesen Stellen schmilzt und das Design somit auf das Transfer-Papier übertragen wird.

Das Transfer-Papier wird anschließend unter Verwendung eines Transfer-Gels, welches auf die Haut aufgetragen wird, an der gewünschten Stelle auf die Haut aufgelegt und angedrückt, sodass das Design oder die Zeichnung auf die Haut übertragen wird.

Die Beschichtung der Stencil-Folie besteht üblicherweise aus einem Wachs/Öl Gemisch, welches wasserlösliche Farbstoffe enthält. Insbesondere eignet sich der Farbstoff Methylviolett (Basic Violet 1, CAS-Nr. 8004-87-3) für diese Anwendung, da er eine starke, langanhaltende Färbung der Haut ermöglicht, sich farblich von den Tattoo-Tinten unterscheidet und auf allen Hauttypen gut sichtbar ist. Durch Inkrafttreten der Verordnung (EU) 2020/2081 der Kommission vom 14. Dezember 2020 betreffend Stoffe in Tätowierfarben oder Permanent-Make-up ("Tätowiermittelverordnung") ist dieser Farbstoff jedoch nicht mehr einsetzbar, nicht nur für Tinten zum Tätowieren, sondern auch für Hilfsmittel dazu, wie eben der Stencil-Folie.

Es ist daher Aufgabe der Erfindung, eine Beschichtung für eine Stencil-Folie zu entwickeln, welche ein farbkräftiges und langanhaltendes Bild auf der Haut erzeugt, insbesondere nach einem der oben beschriebenen Verfahren, welches auf allen Hauttypen gut sichtbar ist. Ferner soll die Beschichtung alle Vorgaben bezüglich der Inhaltsstoffe der Tätowiermittelverordnung EU 2020/2081 und der Verordnung (EG) Nr. 1223/2009 des europäischen Parlamentes und des Rates vom 30. November 2009 über kosmetische Mittel ("Kosmetikverordnung") und der durch Verordnung (EU) 2024/996 der Kommission vom 03. April 2024 geänderten Fassung der Verordnung (EG) Nr. 1223/2009 erfüllen. Darüber hinaus ist es Aufgabe der Erfindung eine Folie mit einer solchen Beschichtung sowie ein Verfahren zur Herstellung der Beschichtung und der Folie anzugeben.

Diese Aufgabe wird gelöst durch eine Beschichtungsmasse, insbesondere eine flüssige Beschichtungsmasse, (oder: Beschichtungsmischung; oder: Wasser-in-Öl-Emulsion) zum Aufbringen auf eine Folie mit den Merkmalen gemäß Anspruch 1. Diese enthält mindestens ein Wachs, wobei das Wachs ausgewählt ist aus einem gemäß EU Verordnung (EG) Nr. 1223/2009 (inklusive der durch Verordnung (EU) 2024/996 geänderten Fassung der Verordnung (EG) Nr. 1223/2009) über kosmetische Mittel zugelassenen Wachs, mindestens einen Farbstoff, wobei der Farbstoff ausgewählt ist aus der Liste der in kosmetischen Mitteln zugelassenen Farbstoffe gemäß EU Verordnung (EG) Nr. 1223/2009 (inklusive der durch Verordnung (EU) 2024/996 geänderten Fassung der Verordnung (EG) Nr. 1223/2009), mindestens einen Emulgator, wobei der Emulgator ausgewählt ist aus einem gemäß EU Verordnung (EG) Nr. 1223/2009 (inklusive der durch Verordnung (EU) 2024/996 geänderten Fassung der Verordnung (EG) Nr. 1223/2009) über kosmetische Mittel zugelassenen Emulgator, mindestens einen pH-Regulator und Wasser.

Gemäß einer bevorzugten Weiterbildung enthält die Beschichtungsmasse ferner mindestens ein Öl, wobei das Öl ausgewählt ist aus einem gemäß EU Verordnung (EG) Nr. 1223/2009 (inklusive der durch Verordnung (EU) 2024/996 geänderten Fassung der Verordnung (EG) Nr. 1223/2009) über kosmetische Mittel zugelassenen Öl. Durch die Verwendung eines Wachs/Öl-Gemisches lässt sich insbesondere der Schmelzpunkt der Beschichtungsmasse variieren. Ferner lässt sich dadurch auch der Übertrag der Masse von der Folie auf das Transfer-papier optimieren.

Bevorzugte in der Beschichtungsmasse enthaltene Wachse und/oder Öle sind hydriertes Kokosglycerid (CAS-Nr. 91744-42-2), Palmöl-Triglycerid (CAS-Nr. 73398-61-5), Polyethylenwachs (CAS-Nr. 9002-88-4), Carnaubawachs (CAS-Nr. 8015-86-6), weißes Mineralöl (CAS-Nr. 8042-47-5), mikrokristallines Paraffinwachs (Cera Microcristallina) (CAS-Nr. 63231-60-7), Reiskleiewachs (rice bran wax) (CAS-Nr. 8016-60-2), Candelilla Wachs (Candelilla Wax) (CAS-Nr. 8006-44-8), Sonnenblumenwachs (Helianthus Annuus Seed Wax) (CAS-Nr. 68937-99-5), C10-C18 Triglyceride (CAS-Nr. 85665-33-4), Squalan (CAS-Nr. 111-01-3) und/oder Paraffinöl (Paraffinum liquidum) (CAS-Nr. 8042-47-5).

Bei einer vorteilhaften Ausführungsform ist das Wachs mit einem Anteil von mindestens 20 Gew.-%, vorzugsweise mindestens 25 Gew.-%, besonders bevorzugt mindestens 30 Gew.-% und/oder einem Anteil von maximal 60 Gew.-%, vorzugsweise maximal 50 Gew.-%, besonders bevorzugt maximal 40 Gew.-% enthalten, und/oder das Öl ist mit einem Anteil von mindestens 10 Gew.-%, vorzugsweise mindestens 15 Gew.-%, besonders bevorzugt mindestens 20 Gew.-% und/oder einem Anteil von maximal 40 Gew.-%, vorzugsweise maximal 35 Gew.-%, besonders bevorzugt maximal 30 Gew.-% enthalten ist. Gehaltsbereiche, die die vorstehend genannten Grenzwerte weder über- oder unterschreiten gewährleisten eine gute Verarbeitbarkeit der Beschichtungsmasse und Aufschmelzverhalten sowohl während der Herstellung als auch der späteren Verwendung der Folie.

Als Farbstoff ist vorzugsweise ein Säurefarbstoff und/oder ein basischer Farbstoff enthalten, insbesondere Acid Red 33 (CI 17200), Acid Red 14 (CI 14720), Food Black 1 (CI 28440) und/oder Brilliant Blau FCF / Acid Blue 9 (CI 42090) enthalten ist. Um einen ausreichend farbkräftigen und langanhalten Übertrag auf der Haut zu erreichen ist der mindestens eine Farbstoff mit einem Anteil von mindestens 5 Gew.-%, vorzugsweise mindestens 9 Gew.-%, besonders bevorzugt mindestens 12 Gew.-% und/oder einem Anteil von maximal 25 Gew.-%, vorzugsweise maximal 21 Gew.-%, besonders bevorzugt maximal 17 Gew.-% enthalten.

Bei einer bevorzugten Ausführungsform ist ein Emulgator aus der Gruppe der nichtionischen, anionischen, kationischen und/oder amphoteren Emulgatoren enthalten. Ferner ist bevorzugt ein Emulgator, der einen HLB-Wert ("Hydrophilic-Lipophilic-Balance") zwischen 6 und 15, vorzugsweise einen HLB-Wert zwischen 10 und 12 aufweist. Solche Emulgatoren werden eigentlich für Öl-in-Wasser-Emulsionen eingesetzt. Es hat sich jedoch überraschenderweise gezeigt, dass diese die besten Ergebnisse hinsichtlich der Verteilung des Wassers und des(r) enthaltenen Farbstoffe(s) erzielen. Es ist essenziell, dass der HLB so gewählt wird, dass sich einerseits der/die Farbstoffe lösen, wofür ein hoher HLB günstig ist, und andererseits eine stabile Wasser-in-Öl-Emulsion entsteht und das erzeugte Bild auf der Haut wischfest ist, wofür ein niedriger HLB günstig ist.

Als geeignete Emulgatoren können in der Beschichtungsmasse Polyalkylenglycolether, Fettsäureester, insbesondere Glycerinstearat und/oder ein Emulgator auf Polyglycerinbasis enthalten sein. Als Polyalkylenglycolether kommt insbesondere ein Diethylenglykolmonooleylether / Oleth-2 zum Einsatz, der unter dem Handelsnamen Nikkol BO-2V (CAS-Nr. 5274-65-7) erhältlich ist. Als Fettsäureester kann vorzugsweise ein Polyglycerylfettsäureester mit einem HLB-Wert von 12, erhältlich unter dem Handelsnamen Nikkol Decaglyn 1-SV (CAS-Nr. 79777-30-3) eingesetzt werden. Unter dem Handelsnamen Gransurf PG-14, (CAS-Nr. 107615-51-0) erhältlich ist ein Emulgator auf Polyglycerinbasis mit einem HLB-Wert von 9 - 9,5. Als bevorzugt hat sich ferner herausgestellt, wenn der mindestens 5 Gew.-%, besonders bevorzugt mindestens 7 Gew.-% und/oder einem Anteil von maximal 20 Gew.-%, vorzugsweise maximal 16 Gew.-%, besonders bevorzugt maximal 12 Gew.-% enthalten ist.

Als pH-Regulator ist gemäß einer vorteilhaften Weiterbildung eine Alkalie, insbesondere Natriumhydroxid (CAS-Nr. 1310-73-2), Kaliumhydroxid (CAS-Nr. 1310-58-3) oder Calciumhydroxid (CAS-Nr. 1305-62-0), enthalten. Bevorzugt ist der pH-Regulator mit einem Anteil von mindestens 0,01 Gew.-%, vorzugsweise mindestens 0,03 Gew.-%, besonders bevorzugt mindestens 0,05 Gew.-% und/oder einem maximalen Anteil kleiner 0,1 Gew.-%, vorzugsweise maximal 0,09 Gew.-%, besonders bevorzugt maximal 0,08 Gew.-% enthalten. Überraschenderweise hat sich gezeigt, dass das Bild auf der Haut wischfester ist, wenn eine Alkalie, z.B. Natriumhydroxid (NaOH) zur pH-Wert-Regulierung in der Rezeptur enthalten ist.

Der restliche Anteil an der Beschichtungsmasse entfällt jeweils auf Wasser, wobei sich hier ein maximaler Anteil von 30 Gew.-%, insbesondere ein maximaler Anteil von 20 Gew.-%, und ein minimaler Anteil von 10 Gew.-%, insbesondere ein minimaler Anteil von 15 Gew.-% als geeignet erwiesen haben. Hinsichtlich der Folie wird die Aufgabe gelöst mit einer Folie, insbesondere einer Stencil-Folie, mit den Merkmalen gemäß Anspruch 9. Die Folie weist eine Beschichtung auf, die durch Aufbringen der zuvor beschriebenen Beschichtungsmasse gebildet ist.

Gemäß einer bevorzugten Ausführungsform weist die Beschichtung eine Schichtdicke zwischen 3 und 25µm, vorzugsweise eine Schichtdicke zwischen 5 und 20µm, besonders bevorzugt eine Schichtdicke zwischen 8 und 15µm, idealerweise 10µm auf.

Die Folie ist insbesondere eine Polymerfolie, vorzugsweise eine PET-Folie oder eine PP-Folie ist.

Insbesondere weist die Folie eine Dicke zwischen 10 und 15µm, vorzugsweise eine Dicke zwischen 11 und 13µm auf.

Hinsichtlich des Verfahrens zur Herstellung einer Beschichtungsmasse, insbesondere einer flüssigen Beschichtungsmasse, wird die Aufgabe gelöst durch ein Verfahren nach Anspruch 13 zur Herstellung einer zuvor beschriebenen Beschichtungsmasse. Das Verfahren umfasst die folgenden Schritte:
a) Aufschmelzen und Homogenisieren des mindestens einen Waches, insbesondere Aufschmelzen und Homogenisieren des mindestens einen Wachses und des mindestens einen Öls bei einer Temperatur zwischen 90 und 110°C zum Erzeugen einer Schmelze,
b) Zugabe und Auflösen des mindestens einen Emulgators in der Schmelze,
c) Zugabe und Dispergieren des mindestens einen Farbstoffes in der Schmelze und Homogenisieren der Schmelze,
d) Auflösen des mindestens einen pH-Regulators in heißem Wasser, insbesondere mit einer Temperatur zwischen 90 und 99°C, und Zugabe des den pH-Regulator enthaltenden heißen Wassers zu der Schmelze,
e) Mischen und Homogenisieren der Schmelze, um die Beschichtungsmasse in Form einer Wasser-in-Öl-Emulsion auszubilden.

Als Wachse wird ein Wachs ausgewählt aus einem gemäß EU Verordnung (EG) Nr. 1223/2009 (inklusive der durch Verordnung (EU) 2024/996 geänderten Fassung der Verordnung (EG) Nr. 1223/2009) über kosmetische Mittel zugelassenen Wachs zugegeben. Als Farbstoff wird ein aus der Liste der in kosmetischen Mitteln zugelassenen Farbstoffe gemäß EU Verordnung (EG) Nr. 1223/2009 (inklusive der durch Verordnung (EU) 2024/996 geänderten Fassung der Verordnung (EG) Nr. 1223/2009) ausgewählter Farbstoff zugegeben. Als Emulgator wird Emulgator ausgewählt aus einem gemäß EU Verordnung (EG) Nr. 1223/2009 (inklusive der durch Verordnung (EU) 2024/996 geänderten Fassung der Verordnung (EG) Nr. 1223/2009) über kosmetische Mittel zugelassenen Emulgator zugegeben.

Als Öl wird insbesondere ein Öl ausgewählt aus einem gemäß EU Verordnung (EG) Nr. 1223/2009 (inklusive der durch Verordnung (EU) 2024/996 geänderten Fassung der Verordnung (EG) Nr. 1223/2009) über kosmetische Mittel zugelassenen Öl zugegeben.

Vorzugsweise werden als Wachs und/oder Öl Öl hydriertes Kokosglycerid (CAS-Nr. 91744-42-2), Palmöl-Triglycerid (CAS-Nr. 73398-61-5), Polyethylenwachs (CAS-Nr. 9002-88-4), Carnaubawachs (CAS-Nr. 8015-86-6), weißes Mineralöl (CAS-Nr. 8042-47-5), mikrokristallines Paraffinwachs (Cera Microcristallina) (CAS-Nr. 63231-60-7), Reiskleiewachs (rice bran wax) (CAS-Nr. 8016-60-2), Candelilla Wachs (Candelilla Wax) (CAS-Nr. 8006-44-8), Sonnenblumenwachs (Helianthus Annuus Seed Wax) (CAS-Nr. 68937-99-5), C10-C18 Triglyceride (CAS-Nr. 85665-33-4), Squalan (CAS-Nr. 111-01-3) und/oder Paraffinöl (Paraffinum liquidum) (CAS-Nr. 8042-47-5 zugegeben.

Das Wachs wird insbesondere derart zugegeben, dass das Wachs mit einem Anteil von mindestens 20 Gew.-%, vorzugsweise mindestens 25 Gew.-%, besonders bevorzugt mindestens 30 Gew.-% und/oder einem Anteil von maximal 60 Gew.-%, vorzugsweise maximal 50 Gew.-%, besonders bevorzugt maximal 40 Gew.-% in der Beschichtungsmasse enthalten ist.

Das Öl wird insbesondere derart zugegeben, dass das Öl mit einem Anteil von mindestens 10 Gew.-%, vorzugsweise mindestens 15 Gew.-%, besonders bevorzugt mindestens 20 Gew.-% und/oder einem Anteil von maximal 40 Gew.-%, vorzugsweise maximal 35 Gew.-%, besonders bevorzugt maximal 30 Gew.-% in der Beschichtungsmasse enthalten ist.

Als Farbstoff wird vorzugsweise ein Säurefarbstoff und/oder ein basischer Farbstoff zugegeben, insbesondere Acid Red 33 (CI 17200), Acid Red 14 (CI 14700), Food Black 1 (CI 28440) und/oder Brilliant Blau FCF / Acid Blue 9 (CI 42090).

Insbesondere wird der Farbstoff derart zugegeben, dass der Farbstoff mit einem Anteil von mindestens 5 Gew.-%, vorzugsweise mindestens 9 Gew.-%, besonders bevorzugt mindestens 12 Gew.-% und/oder einem Anteil von maximal 25 Gew.-%, vorzugsweise maximal 21 Gew.-%, besonders bevorzugt maximal 17 Gew.-% in der Beschichtungsmasse enthalten ist.

Als Emulgator wird vorzugsweise ein Emulgator aus der Gruppe der nichtionischen, anionischen, kationischen und/oder amphoteren Emulgatoren und/oder ein Emulgator mit einem HLB-Wert (Hydrophilic-Lipophilic-Balance) zwischen 6 und 15, vorzugsweise einen HLB-Wert zwischen 10 und 12, zugegeben.

Bevorzugt wird als Emulgator ein Polyalkylenglycolether, ein Fettsäureester, insbesondere ein Polyglycerylfettsäureester und/oder ein Emulgator auf Polyglycerinbasis zugegeben, und/oder der mindestens eine Emulgator wird derart zugegeben, dass dieser mit einem Anteil von mindestens 3 Gew.-%, vorzugsweise mindestens 5 Gew.-%, besonders bevorzugt mindestens 7 Gew.-% und/oder einem Anteil von maximal 20 Gew.-%, vorzugsweise maximal 16 Gew.-%, besonders bevorzugt maximal 12 Gew.-% in der Beschichtungsmasse enthalten ist.

Als pH-Regulator wird vorzugsweise eine Alkalie, insbesondere Natriumhydroxid (CAS-Nr. 1310-73-2), Kaliumhydroxid (CAS-Nr. 1310-58-3) oder Calciumhydroxid (CAS-Nr. 1305-62-0) zugegeben.

Vorzugsweise wird der pH-Regulator derart zugegeben, dass dieser mit einem Anteil von mindestens 0,01 Gew.-%, vorzugsweise mindestens 0,03 Gew.-%, besonders bevorzugt mindestens 0,05 Gew.-% und/oder einem maximalen Anteil kleiner 0,1 Gew.-%, vorzugsweise maximal 0,09 Gew.-%, besonders bevorzugt maximal 0,08 Gew.-% in der Beschichtungsmasse enthalten ist.

Hinsichtlich des Verfahrens zur Herstellung einer Folie wird die Aufgabe gelöst durch ein Verfahren nach Anspruch 14 zur Herstellung einer zuvor beschriebenen Folie. Das Verfahren umfasst die folgenden Schritte:
a) Bereitstellen einer Folie, insbesondere einer Polymerfolie, vorzugsweise einer PET-Folie oder einer PP-Folie, und/oder insbesondere mit einer Dicke zwischen 10 und 15µm, vorzugsweise einer Dicke zwischen 11 und 13µm,
b) Herstellen der Beschichtungsmasse nach einem zuvor beschriebenen Verfahren,
c) Aufbringen der heißen, flüssigen Beschichtungsmasse auf zumindest eine der beiden Flachseiten der Folie und Ausbilden der Beschichtung, insbesondere derart, dass die Beschichtung eine Schichtdicke zwischen 3 und 25µm, vorzugsweise eine Schichtdicke zwischen 5 und 20µm, besonders bevorzugt eine Schichtdicke zwischen 8 und 15µm aufweist.

Das Aufbringen der heißen, flüssigen Beschichtungsmasse erfolgt mittels einer Anlage zur Folienbeschichtung, welche dem Fachmann hinlänglich bekannt sind. Dabei wird die geschmolzene Masse über mehrere Metallzylinder auf die Folie beschichtet und anschließend abgekühlt.

Das Aufbringen der Beschichtungsmasse erfolgt vorzugsweise mit einer Beschichtungsgeschwindigkeit von 100 m/min oder mehr. Überraschenderweise hat sich gezeigt, dass, obwohl die Wachsmischung Wasser enthält, welches beim Aufwickeln der Folie zu einem Verkleben führen könnte, solche Beschichtungs-Geschwindigkeiten von 100 m/min und mehr möglich sind.

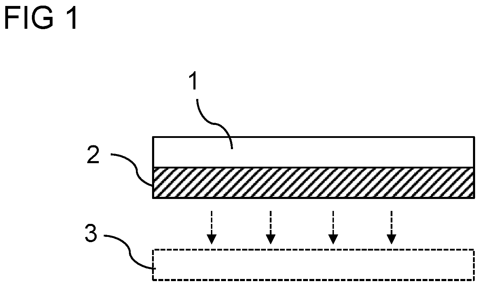

Die Erfindung wird nun nachfolgend anhand von Ausführungsbeispielen und einer Zeichnung (Fig. 1), die eine Folie mit einer Beschichtung zeigt, näher erläutert.

Die Folie 1 ist vorliegend eine Polymerfolie, nämlich eine PP-Folie, mit einer Dicke von 12µm. Die Folie 1 weist eine Beschichtung 2 auf, die durch Aufbringen einer Beschichtungsmasse gebildet und vorliegend auf der unteren Flachseite der Folie 1 ausgebildet ist. Die Beschichtung 2 weist exemplarisch eine Dicke von 12 µm auf. In Fig. 1 gestrichelt angedeutet ist ferner ein Transfer-Papier 3 auf welches das Design bei der Verwendung der Folie 1 als Stencil-Folie übertragen wird, entweder durch Zeichnen auf die - in Fig. 1 - oben liegende, der Beschichtung 2 abgewandte Seite der Folie 1 oder unter Verwendung eines Thermodruckers.

Im Folgenden sind drei Beispielrezepturen für eine Beschichtungsmasse aufgeführt, die auf die Folie 1 aufgebracht werden können und dort die Beschichtung 2 ausbilden. Die Angaben in den Beispielrezepturen sind in Gewichtsprozent und beziehen sich jeweils auf die Gesamtmasse der Beschichtungsmasse bzw. Beschichtungsmischung.

Zur Herstellung der Beschichtungsmasse bzw. Mischung hat sich folgende Vorgehensweise als besonders vorteilhaft herausgestellt: Zunächst wird das Wachs oder Wachs/Öl-Gemisch bei ca. 95°C geschmolzen. Dann wird der Emulgator/die Emulgatoren zugegeben und in der Schmelze gelöst. Anschließend wird/werden der Farbstoff/die Farbstoffe mittels eines Rotor-Stator-Mischers in der Schmelze dispergiert. Anschließend wird heißes Wasser, mit einer Temperatur von ca. 95°C, in dem der pH Regulator gelöst ist, zugegeben und die Komponenten werden weiter mit dem Mischer gemischt und emulgiert. Es hat sich gezeigt, dass dadurch eine sehr homogene und fein verteilte Wasser-in-Öl-Emulsion (W/O-Emulsion) als Beschichtungsmasse hergestellt werden kann.

Überraschenderweise hat sich gezeigt, dass die kurze Zeit (wenige Sekunden), welche dem Wasser bleibt, die Wachs-unlöslichen Farbstoffe zu lösen, bevor es emulgiert wird, ausreicht, um die Farbstoffe sehr fein zu verteilen. Nach dem Mischen kann die heiße, flüssige Mischung mittels einer Anlage zur Folienbeschichtung auf die Folie 1 aufgetragen werden, vorzugsweise mit einer Beschichtungs-Geschwindigkeit von 100 m/min.

### Beispielrezeptur 1:

| | |
|---|---|
| Hydriertes Kokosglycerid (CAS-Nr. 91744-42-2) ¹⁾ | 31,9 |
| Palmöl-Triglycerid (CAS-Nr. 73398-61-5) ²⁾ | 27,8 |
| Nikkol BO-2V (CAS-Nr. 5274-65-7) ³⁾ | 10 |
| Acid Red 33 (CI 117200) ¹⁾ | 13,2 |
| Wasser demin. (CAS-Nr. 7732-18-5) | 17,01 |
| NaOH-Perlen (CAS-Nr. 1310-73-2) | 0,09 |

### Beispielrezeptur 2:

| | |
|---|---|
| Polyethylenwachs (CAS-Nr. 9002-88-4) ⁴⁾ | 38,5 |
| Paraffinum liquidum Ph. Eur. (CAS-Nr. 8042-47-5) ⁵⁾ | 21,2 |
| Gransurf PG-14 (CAS-Nr. 107615-51-0; 33940-99-7) ³⁾ | 7,5 |
| Acid Blue 9 (CI 42090) ⁶⁾ | 8,4 |
| Food Black 1 (CAS-Nr. 2519-30-4) ⁶⁾ | 7,4 |
| Wasser demin. (CAS-Nr. 7732-18-5) | 16,92 |
| NaOH-Perlen (CAS-Nr. 1310-73-2) | 0,0,08 |

### Beispielrezeptur 3:

| | |
|---|---|
| Carnaubawachs (CAS-Nr. 8015-86-6) ⁷⁾ | 35,10 Gew.-% |
| Paraffinum liquidum Ph. Eur. (CAS-Nr. 8042-47-5) ⁵⁾ | 23,33 Gew.-% |
| Nikkol Decaglyn 1-SV (CAS-Nr. 79777-30-3) ³⁾ | 8,80 Gew.-% |
| Brilliant Blau FCF (Acid Blue 9) (CI 42090) ⁶⁾ | 5,80 Gew.-% |
| Acid Red 33 (CI 17200) ¹⁾ | 8,80 Gew.-% |
| Wasser demin. (CAS-Nr. 7732-18-5) | 18,10 Gew.-% |
| NaOH-Perlen, (CAS-Nr. 1310-73-2) | 0,07 Gew.-% |

### Hersteller/Lieferant:

1) Nordmann, Rassmann GmbH, Kajen 2, 20459 Hamburg
2) SysKem Chemie GmbH, Brucknerweg 26, 42289 Wuppertal
3) IMCD Deutschland GmbH, Konrad-Adenauer-Ufer 41-45, 50668 Köln
4) TER Chemicals GmbH & Co. KG, Börsenbrücke 2, 20457 Hamburg
5) Fa. Gustav Heess, Mollenbachstraße 29, 72201 Leonberg
6) Extrachem GmbH, Huchzermeierstraße 8a, 33611 Bielefeld
7) Wachs- und Ceresin Fabriken Th. C. Tromm GmbH, Delmenhorster Straße 4, 50735 Köln

## Patentansprüche

1. Beschichtungsmasse zum Aufbringen auf eine Folie enthaltend
- mindestens ein Wachs, wobei das Wachs ausgewählt ist aus einem gemäß EU Verordnung (EG) Nr. 1223/2009 über kosmetische Mittel zugelassenen Wachs,
- mindestens einen Farbstoff, wobei der Farbstoff ausgewählt ist aus der Liste der in kosmetischen Mitteln zugelassenen Farbstoffe gemäß EU Verordnung (EG) Nr. 1223/2009,
- mindestens einen Emulgator, wobei der Emulgator ausgewählt ist aus einem gemäß EU Verordnung (EG) Nr. 1223/2009 über kosmetische Mittel zugelassenen Emulgator,
- mindestens einen pH-Regulator und
- Wasser.

2. Beschichtungsmasse nach Anspruch 1, ferner enthaltend mindestens ein Öl, wobei das Öl ausgewählt ist aus einem gemäß EU Verordnung (EG) Nr. 1223/2009 über kosmetische Mittel zugelassenen Öl.

3. Beschichtungsmasse nach einem der Ansprüche 1 oder 2, wobei als Wachs und/oder Öl hydriertes Kokosglycerid (CAS-Nr. 91744-42-2), Palmöl-Triglycerid (CAS-Nr. 73398-61-5), Polyethylenwachs (CAS-Nr. 9002-88-4), Carnaubawachs (CAS-Nr. 8015-86-6), weißes Mineralöl (CAS-Nr. 8042-47-5), mikrokristallines Paraffinwachs (Cera Microcristallina) (CAS-Nr. 63231-60-7), Reiskleiewachs (rice bran wax) (CAS-Nr. 8016-60-2), Candelilla Wachs (Candelilla Wax) (CAS-Nr. 8006-44-8), Sonnenblumenwachs (Helianthus Annuus Seed Wax) (CAS-Nr. 68937-99-5), C10-C18 Triglyceride (CAS-Nr. 85665-33-4), Squalan (CAS-Nr. 111-01-3) und/oder Paraffinöl (Paraffinum liquidum) (CAS-Nr. 8042-47-5 enthalten ist.

4. Beschichtungsmasse nach einem der vorhergehenden Ansprüche, wobei das Wachs mit einem Anteil von mindestens 20 Gew.-%, vorzugsweise mindestens 25 Gew.-%, besonders bevorzugt mindestens 30 Gew.-% und/oder einem Anteil von maximal 60 Gew.-%, vorzugsweise maximal 50 Gew.-%, besonders bevorzugt maximal 40 Gew.-% enthalten ist, und/oder
wobei das Öl mit einem Anteil von mindestens 10 Gew.-%, vorzugsweise mindestens 15 Gew.-%, besonders bevorzugt mindestens 20 Gew.-% und/oder einem Anteil von maximal 40 Gew.-%, vorzugsweise maximal 35 Gew.-%, besonders bevorzugt maximal 30 Gew.-% enthalten ist.

5. Beschichtungsmasse nach einem der vorhergehenden Ansprüche, wobei als Farbstoff ein Säurefarbstoff und/oder ein basischer Farbstoff enthalten ist, insbesondere Acid Red 33 (CI 17200), Acid Red 14 (CI 14720), Food Black 1 (CI 28440) und/oder Brilliant Blau FCF / Acid Blue 9 (CI 42090) enthalten ist, und/oder
wobei der mindestens eine Farbstoff mit einem Anteil von mindestens 5 Gew.-%, vorzugsweise mindestens 9 Gew.-%, besonders bevorzugt mindestens 12 Gew.-% und/oder einem Anteil von maximal 25 Gew.-%, vorzugsweise maximal 21 Gew.-%, besonders bevorzugt maximal 17 Gew.-% enthalten ist.

6. Beschichtungsmasse nach einem der vorhergehenden Ansprüche, wobei ein Emulgator aus der Gruppe der nichtionischen, anionischen, kationischen und/oder amphoteren Emulgatoren enthalten ist und/oder wobei der Emulgator einen HLB-Wert ((Hydrophilic-Lipophilic-Balance) zwischen 6 und 15, vorzugsweise einen HLB-Wert zwischen 10 und 12 aufweist.

7. Beschichtungsmasse nach einem der vorhergehenden Ansprüche, wobei als Emulgator ein Polyalkylenglycolether, ein Fettsäureester, insbesondere ein Polyglycerylfettsäureester und/oder ein Emulgator auf Polyglycerinbasis enthalten ist, und/oder
wobei der mindestens eine Emulgator mit einem Anteil von mindestens 3 Gew.-%, vorzugsweise mindestens 5 Gew.-%, besonders bevorzugt mindestens 7 Gew.-% und/oder einem Anteil von maximal 20 Gew.-%, vorzugsweise maximal 16 Gew.-%, besonders bevorzugt maximal 12 Gew.-% enthalten ist.

8. Beschichtungsmasse nach einem der vorhergehenden Ansprüche, wobei als pH-Regulator eine Alkalie, insbesondere Natriumhydroxid (CAS-Nr. 1310-73-2), Kaliumhydroxid (CAS-Nr. 1310-58-3) oder Calciumhydroxid (CAS-Nr. 1305-62-0) enthalten ist, und/oder
wobei der mindestens eine pH-Regulator mit einem Anteil von mindestens 0,01 Gew.-%, vorzugsweise mindestens 0,03 Gew.-%, besonders bevorzugt mindestens 0,05 Gew.-% und/oder einem maximalen Anteil kleiner 0,1 Gew.-%, vorzugsweise maximal 0,09 Gew.-%, besonders bevorzugt maximal 0,08 Gew.-% enthalten ist.

9. Folie mit einer Beschichtung, wobei die Beschichtung durch Aufbringen einer Beschichtungsmasse nach einem der vorhergehenden Ansprüche auf zumindest eine der beiden Flachseiten der Folie gebildet ist.

10. Folie nach Anspruch 9, wobei die Beschichtung eine Schichtdicke zwischen 3 und 25µm, vorzugsweise eine Schichtdicke zwischen 5 und 20µm, besonders bevorzugt eine Schichtdicke zwischen 8 und 15µm aufweist.

11. Folie nach einem der Ansprüche 9 oder 10, wobei die Folie eine Polymerfolie, vorzugsweise eine PET-Folie oder eine PP-Folie ist.

12. Folie nach einem der Ansprüche 9 bis 11, wobei die Folie eine Dicke zwischen 10 und 15µm, vorzugsweise eine Dicke zwischen 11 und 13µm aufweist.

13. Verfahren zur Herstellung einer Beschichtungsmasse nach einem der Ansprüche 1 bis 8, umfassend die folgenden Schritte:
a) Aufschmelzen und Homogenisieren des mindestens einen Wachses, insbesondere Aufschmelzen und Homogenisieren des mindestens einen Wachses und des mindestens einen Öls bei einer Temperatur zwischen 90 und 110°C zum Erzeugen einer Schmelze,
b) Zugabe und Auflösen des mindestens einen Emulgators in der Schmelze,
c) Zugabe und Dispergieren des mindestens einen Farbstoffes und Homogenisieren der Schmelze,
d) Auflösen des mindestens einen pH-Regulators in heißem Wasser, insbesondere mit einer Temperatur zwischen 90 und 99°C, und Zugabe des den pH-Regulator enthaltenden Wassers zu der Schmelze,
e) Mischen und Homogenisieren der Schmelze.

14. Verfahren zur Herstellung einer Folie nach einem der Ansprüche 9 bis 12, umfassend die folgenden Schritte:
a) Bereitstellen einer Folie, insbesondere einer Polymerfolie, vorzugsweise einer PET-Folie oder einer PP-Folie, und/oder insbesondere mit einer Dicke zwischen 10 und 15µm, vorzugsweise einer Dicke zwischen 11 und 13µm,
b) Herstellen der Beschichtungsmasse nach einem Verfahren nach Anspruch 13,
c) Aufbringen der Beschichtungsmasse auf zumindest eine der beiden Flachseiten der Folie und Ausbilden der Beschichtung, insbesondere derart, dass die Beschichtung eine Schichtdicke zwischen 3 und 25µm, vorzugsweise eine Schichtdicke zwischen 5 und 20µm, besonders bevorzugt eine Schichtdicke zwischen 8 und 15µm aufweist.

15. Verfahren nach Anspruch 14, wobei das Aufbringen der Beschichtungsmasse mit einer Beschichtungsgeschwindigkeit von 100 m/min oder mehr erfolgt.

## Geänderte Patentansprüche

### Geänderte Patentansprüche gemäss Regel 137(2) EPÜ.

1. Beschichtungsmasse zum Aufbringen auf eine Folie enthaltend
- mindestens ein Wachs, wobei das Wachs mit einem Anteil von mindestens 20 Gew.-% und einem Anteil von maximal 60 Gew.-% enthalten ist,
- mindestens ein Öl, wobei das Öl mit einem Anteil von mindestens 10 Gew.-% und einem Anteil von maximal 40 Gew.-% enthalten ist, und wobei als Wachs oder Öl hydriertes Kokosglycerid (CAS-Nr. 91744-42-2), Palmöl-Triglycerid (CAS-Nr. 73398-61-5), Polyethylenwachs (CAS-Nr. 9002-88-4), Carnaubawachs (CAS-Nr. 8015-86-6), weißes Mineralöl (CAS-Nr. 8042-47-5), mikrokristallines Paraffinwachs (Cera Microcristallina) (CAS-Nr. 63231-60-7), Reiskleiewachs (rice bran wax) (CAS-Nr. 8016-60-2), Candelilla Wachs (Candelilla Wax) (CAS-Nr. 8006-44-8), Sonnenblumenwachs (Helianthus Annuus Seed Wax) (CAS-Nr. 68937-99-5), C10-C18 Triglyceride (CAS-Nr. 85665-33-4), Squalan (CAS-Nr. 111-01-3) und/oder Paraffinöl (Paraffinum liquidum) (CAS-Nr. 8042-47-5 enthalten ist,
- mindestens einen Farbstoff, wobei als Farbstoff ein Säurefarbstoff und/oder ein basischer Farbstoff enthalten ist, ,
- mindestens einen Emulgator, wobei ein Emulgator aus der Gruppe der nichtionischen, anionischen, kationischen und/oder amphoteren Emulgatoren enthalten ist,
- mindestens einen pH-Regulator und
- Wasser.

2. Beschichtungsmasse nach einem der vorhergehenden Ansprüche,
wobei das Wachs mit einem Anteil von mindestens 25 Gew.-%, vorzugsweise mindestens 30 Gew.-% und einem Anteil von maximal 50 Gew.-%, vorzugsweise maximal 40 Gew.-% enthalten ist, und/oder wobei das Öl mit einem Anteil von mindestens 15 Gew.-%, vorzugsweise mindestens 20 Gew.-% und einem Anteil von maximal 35 Gew.-%, vorzugsweise maximal 30 Gew.-% enthalten ist.

3. Beschichtungsmasse nach einem der vorhergehenden Ansprüche, wobei als Farbstoff Acid Red 33 (CI 17200), Acid Red 14 (CI 14720), Food Black 1 (CI 28440) und/oder Brilliant Blau FCF / Acid Blue 9 (CI 42090) enthalten ist, und/oder
wobei der mindestens eine Farbstoff mit einem Anteil von mindestens 5 Gew.-%, vorzugsweise mindestens 9 Gew.-%, besonders bevorzugt mindestens 12 Gew.-% und einem Anteil von maximal 25 Gew.-%, vorzugsweise maximal 21 Gew.-%, besonders bevorzugt maximal 17 Gew.-% enthalten ist.

4. Beschichtungsmasse nach einem der vorhergehenden Ansprüche wobei der Emulgator einen HLB-Wert ((Hydrophilic-Lipophilic-Balance) zwischen 6 und 15, vorzugsweise einen HLB-Wert zwischen 10 und 12 aufweist.

5. Beschichtungsmasse nach einem der vorhergehenden Ansprüche, wobei als Emulgator ein Polyalkylenglycolether, ein Fettsäureester, insbesondere ein Polyglycerylfettsäureester und/oder ein Emulgator auf Polyglycerinbasis enthalten ist, und/oder
wobei der mindestens eine Emulgator mit einem Anteil von mindestens 3 Gew.-%, vorzugsweise mindestens 5 Gew.-%, besonders bevorzugt mindestens 7 Gew.-% und einem Anteil von maximal 20 Gew.-%, vorzugsweise maximal 16 Gew.-%, besonders bevorzugt maximal 12 Gew.-% enthalten ist.

6. Beschichtungsmasse nach einem der vorhergehenden Ansprüche, wobei als pH-Regulator eine Alkalie, insbesondere Natriumhydroxid (CAS-Nr. 1310-73-2), Kaliumhydroxid (CAS-Nr. 1310-58-3) oder Calciumhydroxid (CAS-Nr. 1305-62-0) enthalten ist, und/oder
wobei der mindestens eine pH-Regulator mit einem Anteil von mindestens 0,01 Gew.-%, vorzugsweise mindestens 0,03 Gew.-%, besonders bevorzugt mindestens 0,05 Gew.-% und einen Anteil von maximal 0,1 Gew.-%, vorzugsweise maximal 0,09 Gew.-%, besonders bevorzugt maximal 0,08 Gew.-% enthalten ist.

7. Folie mit einer Beschichtung aus einer Beschichtungsmasse nach einem der vorhergehenden Ansprüche, wobei die Beschichtung durch Aufbringen der Beschichtungsmasse auf zumindest eine der beiden Flachseiten der Folie gebildet ist.

8. Folie nach Anspruch 7, wobei die Beschichtung eine Schichtdicke zwischen 3 und 25µm, vorzugsweise eine Schichtdicke zwischen 5 und 20µm, besonders bevorzugt eine Schichtdicke zwischen 8 und 15µm aufweist.

9. Folie nach einem der Ansprüche 7 oder 8, wobei die Folie eine Polymerfolie, vorzugsweise eine PET-Folie oder eine PP-Folie ist.

10. Folie nach einem der Ansprüche 7 bis 9, wobei die Folie eine Dicke zwischen 10 und 15µm, vorzugsweise eine Dicke zwischen 11 und 13µm aufweist.

11. Verfahren zur Herstellung einer Beschichtungsmasse nach einem der Ansprüche 1 bis 6, umfassend die folgenden Schritte:
a) Aufschmelzen und Homogenisieren des mindestens einen Wachses, insbesondere Aufschmelzen und Homogenisieren des mindestens einen Wachses und des mindestens einen Öls bei einer Temperatur zwischen 90 und 110°C zum Erzeugen einer Schmelze,
b) Zugabe und Auflösen des mindestens einen Emulgators in der Schmelze,
c) Zugabe und Dispergieren des mindestens einen Farbstoffes und Homogenisieren der Schmelze,
d) Auflösen des mindestens einen pH-Regulators in heißem Wasser, insbesondere mit einer Temperatur zwischen 90 und 99°C, und Zugabe des den pH-Regulator enthaltenden Wassers zu der Schmelze,
e) Mischen und Homogenisieren der Schmelze.

12. Verfahren zur Herstellung einer Folie nach einem der Ansprüche 7 bis 10, umfassend die folgenden Schritte:
a) Bereitstellen einer Folie, insbesondere einer Polymerfolie, vorzugsweise einer PET-Folie oder einer PP-Folie, und/oder insbesondere mit einer Dicke zwischen 10 und 15µm, vorzugsweise einer Dicke zwischen 11 und 13µm,
b) Herstellen der Beschichtungsmasse nach einem Verfahren nach Anspruch 11,
c) Aufbringen der Beschichtungsmasse auf zumindest eine der beiden Flachseiten der Folie und Ausbilden der Beschichtung, insbesondere derart, dass die Beschichtung eine Schichtdicke zwischen 3 und 25µm, vorzugsweise eine Schichtdicke zwischen 5 und 20µm, besonders bevorzugt eine Schichtdicke zwischen 8 und 15µm aufweist.

13. Verfahren nach Anspruch 12, wobei das Aufbringen der Beschichtungsmasse mit einer Beschichtungsgeschwindigkeit von 100 m/min oder mehr erfolgt.
